# EUROPEAN PATENT APPLICATION

(11) **EP 4 778 446 A1**
(43) Date of publication of application: **22.07.2026**
(21) Application number: 24865453.5
(22) Date of filing: 10.09.2024
(51) Int. Cl.: A61B 3/103, A61B 3/14, G06T 7/00

(54) **INFORMATION PROCESSING SYSTEM, INFORMATION PROCESSING METHOD, AND PROGRAM**

(30) Priority: 13.09.2023 JP 2023148606
(71) Applicant: Innojin, Inc., Tokyo 113-0033 (JP); Juntendo Educational Foundation, Tokyo 113-8421 (JP)
(72) Inventor: INOMATA Takenori, Tokyo 113-0033 (JP); OKUMURA Yuichi, Tokyo 113-0033 (JP)
(74) Representative: Osterhoff, Utz
(86) International application number: PCT/JP2024/032421
(87) International publication number: WO 2025/057953

(57) **Abstract**

[Problem] To enable easy measurement of refractive power.

[Solution] An information processing system comprising: a trained model storage unit that stores a trained model obtained by machine learning using images obtained by photographing an eye including reflected light of the light on the cornea in a state where a light is emitted and irradiated onto the cornea and refraction values of the eye; an image acquisition unit that acquires a photographed image in which a user terminal emits a light and photographs an eye of a user; and an estimation processing unit that provides to the trained model the acquired photographed image to estimate a refraction value of the user.

## Description

### Technical Field

The present invention relates to an information processing system, an information processing method, and a program.

### Background Art

PTL 1 measures the refractive power of an eye using photorefraction.

### Citation List

### Patent Literature

[PTL1] U.S. Patent No. 9408535

### Summary of Invention

### Technical Problem

To provide a technology capable of more easily measuring refractive power than conventional devices for measuring ocular refractive power.

### Solution to Problem

According to a main aspect of the present invention, there is provided an information processing system comprising: a trained model storage unit configured to store a trained model trained by machine learning using an image of an eye captured while light is emitted and irradiated onto a cornea and a refraction value of the eye; an image acquisition unit configured to acquire a captured image of a user's eye obtained by causing a user terminal to emit light; and an estimation processing unit configured to input the acquired captured image to the trained model and estimate a refraction value of the user.

Other problems disclosed by the present application and solutions thereof are clarified by the Description of Embodiments section and the drawings.

### [Advantageous Effects of Invention]

According to the present invention, refractive power can be measured easily.

### Brief Description of Drawings

[Fig. 1] Fig. 1 is a diagram showing an overall configuration example of a refractive power measurement system that is an information processing system according to an embodiment of the present invention.
[Fig. 2] Fig. 2 is a diagram for explaining a state of measurement in the present embodiment.
[Fig. 3] Fig. 3 is a diagram for explaining an eye 20 of a user included in a photographed image.
[Fig. 4] Fig. 4 is a diagram showing a hardware configuration example of a management server 2.
[Fig. 5] Fig. 5 is a diagram showing a software configuration example of the management server 2.
[Fig. 6] Fig. 6 is a diagram for explaining a flow of a training process.
[Fig. 7] Fig. 7 is a diagram for explaining a flow of a prediction process of a refraction value.

### Description of Embodiments

### Summary of Invention

The contents of an embodiment of the present invention will be described by listing. The present invention comprises, for example, the following configurations.

### Item 1

An information processing system comprising:
a trained model storage unit configured to store a trained model trained by machine learning using (i) an image of an eye captured while light is emitted and irradiated onto a cornea and (ii) a refraction value of the eye;
an image acquisition unit configured to acquire a captured image of a user's eye obtained by causing a user terminal to emit light; and
an estimation processing unit configured to input the acquired captured image to the trained model and estimate a refraction value of the user.

### Item 2

The information processing system according to Item 1, characterized in that:
the refraction value includes at least one of spherical power, cylindrical power, and axis.

### Item 3

The information processing system according to Item 1, characterized in that:
the trained model is obtained by machine learning using a region in the image in which reflected light of the light source is captured and the refraction value;
the information processing system comprises a light extraction unit that extracts a light region in which reflected light of the light source is captured from the photographed image; and
the estimation processing unit provides the light region to the trained model to estimate the refraction value.

### Item 4

The information processing system according to Item 1, characterized in that:
the trained model is obtained by machine learning using a region of the cornea included in the image photographed such that the light is incident on the cornea and the refraction value;
the information processing system comprises a cornea extraction unit that extracts a cornea region in which a cornea is shown from the photographed image; and the estimation processing unit provides the cornea region to the trained model to estimate the refraction value.

### Item 5

An information processing method comprising:
by a computer storing a trained model trained by machine learning using (i) an image of a human eye captured while light is emitted and irradiated onto a cornea and (ii) a refraction value of the eye,
acquiring a captured image of a user's eye obtained by causing a user terminal to emit light; and
inputting the acquired captured image to the trained model to estimate a refraction value of the user.

### Item 6

A program for causing a computer to:
store a trained model trained by machine learning using (i) an image of a human eye captured while light is emitted and irradiated onto a cornea and (ii) a refraction value of the eye;
acquire a captured image of a user's eye obtained by causing a user terminal to emit light; and
input the acquired captured image to the trained model to estimate a refraction value of the user.

### Overview of System

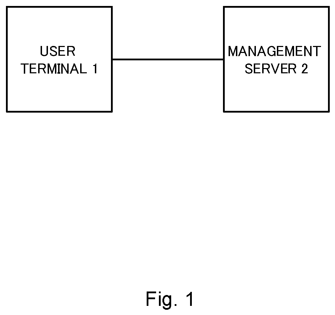

Fig. 1 is a diagram showing an example of an overall configuration of a refractive power measurement system which is an information processing system according to an embodiment of the present invention. The refractive power measurement system of the present embodiment comprises a management server 2. The management server 2 is communicably connected to a user terminal 1 via a communication network. The communication network is, for example, the Internet, and is constructed by a public telephone network, a mobile telephone network, a wireless communication channel, Ethernet (registered trademark), or the like.

The user terminal 1 is a computer operated by a user. The user terminal 1 can be, for example, a smartphone, a tablet computer, a personal computer, or the like. In the present embodiment, it is assumed that the user terminal 1 is a smartphone or a tablet computer, and comprises a camera (not shown) and a flash light (not shown).

The management server 2 may be a general-purpose computer such as a workstation or a personal computer, or may be logically realized by cloud computing.

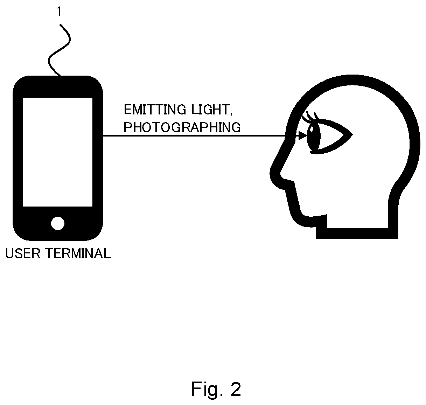

The refractive power measurement system of the present embodiment estimates refractive power of a user's eye. Fig. 2 is a diagram explaining a state of measurement in the present embodiment. In the refractive power measurement system of the present embodiment, a flash light of the user terminal 1 is emitted, light from the flash light is irradiated onto the cornea (the black-appearing part of the eye), and a user (which may be an upper body, may be a face, or may be only an eye part) is photographed by a camera of the user terminal 1, so that light reflected by the cornea is included in a photographed image.

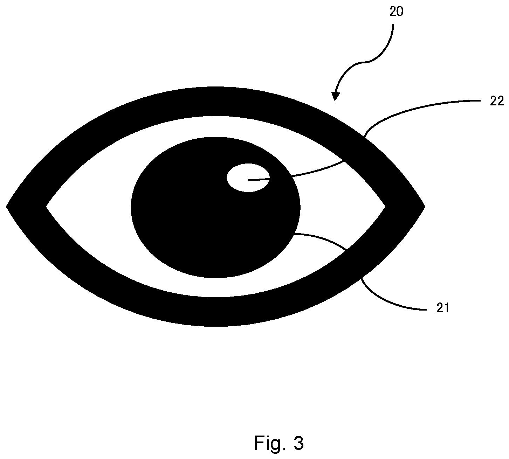

Fig. 3 is a diagram explaining an eye 20 of a user included in a photographed image. Reflected light 22 of a flash light is shown in a cornea 21 in the eye 20. It is assumed that a shape of the flash light is circular. The reflected light 22 may deform into an elliptical shape as shown in Fig. 3. This deformation state changes according to a refraction value.

In the refractive power measurement system of the present embodiment, a value (refraction value) indicating refractive power of a user's eye is measured in advance by a general measurement device. With a general measurement device, for example, a refraction value can be measured by irradiating the eye with light and detecting light reflected from the fundus by a photorefraction method. A trained model is created by machine learning using a refraction value measured by a general measurement device and an image of reflected light from a cornea 21. The image of reflected light may be an image of an entire body of a user, an image photographed of an upper body of a user, an image photographed of a face of a user, or an image photographed of an eye part of a user. Further, the image of reflected light may be obtained by extracting an eye part or a cornea part from an image photographed of a user. In the refractive power measurement system, a refraction value of a user can be estimated by providing a photographed image in which a user photographs himself/herself in a state where a flash light is emitted toward his/her own eye to a trained model.

### Management Server

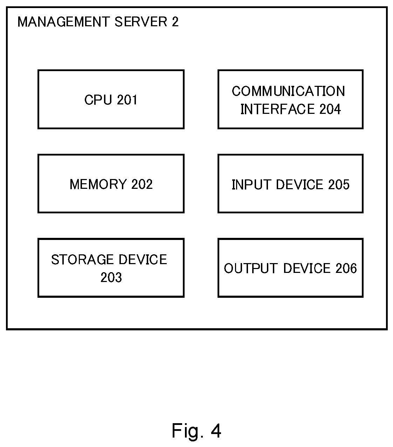

Fig. 4 is a diagram showing an example of a hardware configuration of the management server 2. The illustrated configuration is an example, and configurations other than this may be included. The management server 2 comprises a CPU 201, a memory 202, a storage device 203, a communication interface 204, an input device 205, and an output device 206. The storage device 203 is, for example, a hard disk drive, a solid state drive, a flash memory, or the like that stores various data and programs. The communication interface 204 is an interface for connecting to a communication network, and is, for example, an adapter for connecting to Ethernet (registered trademark), a modem for connecting to a public telephone network, a wireless communication device for performing wireless communication, a USB (Universal Serial Bus) connector or an RS232C connector for serial communication, or the like. The input device 205 is, for example, a keyboard, a mouse, a touch panel, a button, a microphone, or the like that inputs data. The output device 206 is, for example, a display, a printer, a speaker, or the like that outputs data. Each functional unit of the management server 2 described later is realized by the CPU 201 reading a program stored in the storage device 203 into the memory 202 and executing the program, and each storage unit of the management server 2 is realized as a part of a storage area provided by the memory 202 and the storage device 203.

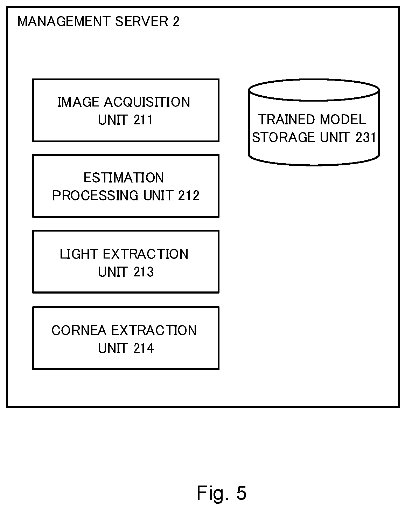

Fig. 5 is a diagram showing an example of a software configuration of the management server 2. The management server 2 comprises a trained model storage unit 231, an image acquisition unit 211, an estimation processing unit 212, a light extraction unit 213, and a cornea extraction unit 214.

### Storage Unit

The trained model storage unit 231 stores a trained model obtained by machine learning using a photographed image of a human and a refraction value of an eye of the human. As described above, a photographed image is an image photographed such that reflected light from a cornea is included in a photographed image when a flash light of the user terminal 1 is emitted, light from the flash light is irradiated onto the cornea of the human, and then the human is photographed by a camera of the user terminal 1. The refraction value of an eye includes at least one of spherical power, cylindrical power, and axis.

The trained model may be created by machine learning using a region of reflected light of a flash light included in a photographed image (hereinafter referred to as a light region) and a refraction value. Further, the trained model may be created by machine learning using a region of a cornea included in a photographed image (hereinafter referred to as a cornea region) and a refraction value.

### Functional Units

The image acquisition unit 211 acquires a photographed image in which the user terminal 1 emits a flash light to photograph a user.

The estimation processing unit 212 provides an acquired photographed image to a trained model to estimate a refraction value of a user.

The light extraction unit 213 can extract a light region in which a flash light is shown from a photographed image. The estimation processing unit 212 can provide a light region to a trained model to estimate a refraction value.

The cornea extraction unit 214 can extract a cornea region in which a cornea is shown from a photographed image. The estimation processing unit 212 can provide a cornea region to a trained model to estimate a refraction value. When the cornea extraction unit 214 cannot extract a cornea region from a photographed image (for example, when eyes are closed), the image acquisition unit 211 may transmit a message instructing to photograph again to the user terminal 1 to reacquire a photographed image.

The light extraction unit 213 may extract a light region from a cornea region of a photographed image. When the light extraction unit 213 cannot extract a light region from a photographed image (for example, when a flash light is not emitted or when light of a flash light is not irradiated onto the cornea region), the image acquisition unit 211 may transmit a message instructing to photograph again to the user terminal 1 to reacquire a photographed image.

### Operation

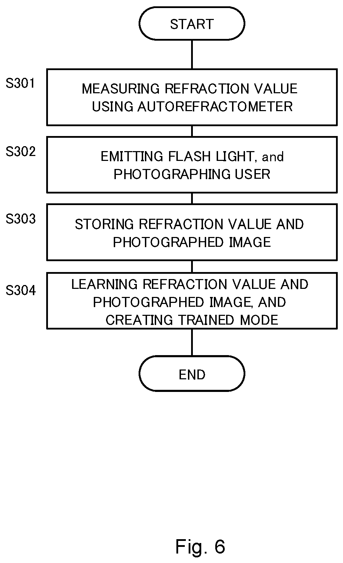

Fig. 6 is a diagram explaining a flow of learning processing. The creation of a trained model may be performed by the user terminal 1 or the management server 2, or may be performed by another computer.

First, a refraction value of a subject is measured using a general measurement device such as an autorefractometer (S301). Next, a flash light of the user terminal 1 (smartphone) is emitted to irradiate an eye of a subject, and the subject is photographed by a camera of the user terminal 1 (S302). A measured refraction value and a photographed image are associated with each other and stored in a computer (S303), and the computer learns a pair of the refraction value and the photographed image by machine learning to create a trained model that infers a refraction value from a photographed image (S304).

A trained model created in this manner is stored in the trained model storage unit 231 of the management server 2.

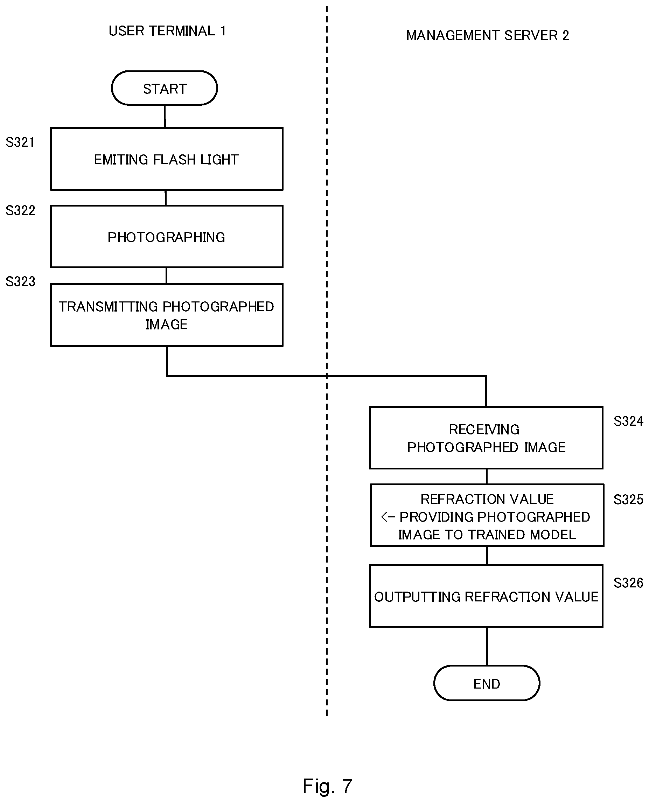

Fig. 7 is a diagram explaining a flow of prediction processing of a refraction value.

The user terminal 1 emits a flash light (S321), photographs a user (S322), and transmits a photographed image to the management server 2 (S323). Here, it is assumed that the user terminal 1 is positioned such that light irradiated from a flash light is reflected by a cornea and reflected light is shown in a photographed image.

The management server 2 receives a photographed image from the user terminal 1 (S324), provides a received photographed image to a trained model stored in the trained model storage unit 231 to infer a refraction value (S325), and can output a refraction value (for example, transmit a refraction value to the user terminal 1 to display or transmit a refraction value to a terminal of a doctor to display) (S326). An image in which an eye or a cornea part is extracted from a photographed image may be provided to a trained model.

The management server 2 may infer a refraction value by specifying a cornea region from a received photographed image and providing an extracted image in which a cornea region part is extracted from a photographed image or a photographed image and information specifying a cornea region to a trained model. Further, the management server 2 may infer a refraction value by specifying a light region from a received photographed image and providing an extracted image in which a light region part is extracted from a photographed image or a photographed image and information specifying a light region to a trained model.

As described above, according to the refractive power measurement system of the present embodiment, a refraction value of a user can be estimated by using a user terminal 1 such as a smartphone, emitting a flash light to photograph a user, and simply providing a photographed image to a trained model. Therefore, refractive power of a user can be easily measured.

Although the present embodiment has been described above, the above embodiment is for facilitating understanding of the present invention and is not for limiting interpretation of the present invention. The present invention can be modified and improved without departing from its spirit, and the present invention also includes equivalents thereof.

For example, in the present embodiment, a flash light is irradiated on an eye of a user, but the present invention is not limited thereto, and for example, a display of the user terminal 1 may emit light to photograph a user using a front-facing camera (not shown). In this case, a guide for alignment of a position of a face (or an eye) of a user can be displayed on a display of the user terminal 1 to fix a position of a face (or an eye). The user terminal 1 can irradiate this light on an eye of a user by controlling to emit a part of a display in bright white light.

Further, in the present embodiment, a shape of a flash light is circular, but for example, the flash light may emit light in a cross shape. For example, a bright white cross can be displayed on a display, alignment can be performed such that this cross enters within a cornea, and then a user can be photographed using a front-facing camera.

### Reference Signs List

- 1: user terminal
- 2: management server

## Claims

1. An information processing system comprising:
a trained model storage unit that stores a trained model obtained by machine learning on an image obtained by photographing an eye including reflected light of the light onto the cornea in a state where the light is emitted and irradiated onto the cornea and a refraction value of the eye;
an image acquisition unit that acquires a photographed image obtained by photographing an eye of a user in a state where a user terminal emits a light and irradiates the light onto a cornea of the user;
and an estimation processing unit that provides the acquired photographed image to the trained model to estimate a refraction value of the user.

2. The information processing system according to Claim 1, wherein
the refraction value includes at least one of a spherical power, cylindrical power, and axis.

3. The information processing system according to Claim 1, wherein
the trained model is obtained by machine learning on a region in which reflected light of the source is captured, the region being included in the image, and the refraction value,
the information processing system comprises a light extraction unit that extracts a region in which a reflected light of the light source is captured from the photographed image,
and the estimation processing unit provides the extracted region to the trained model to estimate the refraction value.

4. The information processing system according to Claim 1, wherein
the trained model is obtained by machine learning on a region of the cornea included in the image photographed such that the light is incident on the cornea and the refraction value,
the information processing system comprises a cornea extraction unit that extracts a cornea region in which a cornea is captured from the photographed image,
and the estimation processing unit provides the cornea region to the trained model to estimate the refraction value.

5. An information processing method executed by a computer comprising:
a step of acquiring a photographed image obtained by photographing an eye of a user in a state where a user terminal emits a light and irradiates the light onto a cornea of the user;
and a step of providing the acquired photographed image to a trained model obtained by machine learning on an image obtained by photographing an eye including reflected light of the light onto the cornea in a state where the light is emitted and irradiated onto the cornea and a refraction value of the eye, to estimate a refraction value of the user.

6. A program for causing a computer to execute:
a step of acquiring a photographed image obtained by photographing an eye of a user in a state where a user terminal emits a light and irradiates the light onto a cornea of the user;
and a step of providing the acquired photographed image to a trained model obtained by machine learning on an image obtained by photographing an eye including reflected light of the light onto the cornea in a state where the light is emitted and irradiated onto the cornea and a refraction value of the eye, to estimate a refraction value of the user.
